# EUROPÄISCHE PATENTSCHRIFT

(11) **EP 3 725 358 B1**
(45) Veröffentlichungstag und Bekanntmachung des Hinweises auf die Patenterteilung: **13.09.2023**
(21) Anmeldenummer: 19169338.1
(22) Anmeldetag: 15.04.2019
(51) Int. Cl.: A61M 37/00, F16H 25/08, F16H 23/02, A01K 11/00

(54) **ANTRIEBSSYSTEM UND ANTRIEBSMODUL FÜR EIN HANDGERÄT ZUM LOKALEN AUFSTECHEN EINER MENSCHLICHEN ODER EINER TIERISCHEN HAUT SOWIE HANDGERÄT**
HAND-HELD DEVICE AND DRIVE SYSTEM AND DRIVE MODULE FOR A HAND-HELD DEVICE FOR LOCAL PIERCING OF HUMAN OR ANIMAL SKIN
SYSTÈME D'ENTRAÎNEMENT ET MODULE D'ENTRAÎNEMENT POUR UN APPAREIL PORTATIF DESTINÉ AU PERCEMENT LOCAL D'UNE PEAU HUMAINE OU ANIMALE AINSI QU'APPAREIL PORTATIF

(43) Veröffentlichungstag der Anmeldung: 21.10.2020
(73) Patentinhaber: MT.DERM GmbH, 14167 Berlin (DE)
(72) Erfinder: SCHERKOWSKI, Dirk, 12489 Berlin (DE)
(74) Vertreter: Bittner, Thomas L.

(56) Entgegenhaltungen:
- EP-A1- 1 495 782
- EP-A1- 1 958 659
- DE-U1-202006 013 148
- US-A1- 2012 123 462
- US-A1- 2018 369 553

## Beschreibung

Die Erfindung betrifft ein Antriebssystem und ein Antriebsmodul für ein Handgerät zum lokalen Aufstechen einer menschlichen oder einer tierischen Haut sowie ein Handgerät.

### Hintergrund

Derartige Hautstechvorrichtungen werden genutzt, um die Haut lokal aufzustechen. Hierbei kann vorgesehen sein, mittels des lokalen Aufstechens der Haut eine Substanz in die Haut einzutragen, beispielsweise einen Farbstoff, in Verbindung mit dem Ausbilden von Tattoos oder Permanentmakeup, oder einen kosmetischen oder medizinischen Stoff.

Übliche Hautstechvorrichtungen sind als Handgerät ausgebildet, bei denen ein Antriebsmodul und ein hieran koppelndes Nadelmodul vorgesehen sind. Das Antriebsmodul weist zum Beispiel einen Elektromotor auf, mit dem eine Antriebsbewegung bereitgestellt wird, um eine Stechnadel durch eine vorderseitige Gehäuseöffnung des Nadelmoduls aus- und einzufahren. Die von dem Antriebsmodul bereitgestellte Antriebsbewegung wird auf die Stechnadel eingekoppelt, sodass diese zum lokalen Aufstechen der Haut vor- und zurückgefahren werden kann. Antriebsmodul und Nadelmodul können lösbar miteinander gekoppelt sein, wobei das Nadelmodul als ein Einwegmodul ausgeführt sein kann. Das Vor- und Zurückbewegen der Stechnadel findet im Betrieb mit einer maschinengesteuerten Wiederholfrequenz statt.

Das Dokument DE 20 2006 013148 U1 betrifft eine Vorrichtung zur Übertragung von axial gerichteten Kräften auf Nadeln einer Tätowier- oder Schminkmaschine, umfassend einen Pleuel, welcher eine axial gerichtete Kraft auf mindestens eine Nadel überträgt, dadurch gekennzeichnet, dass sie ein Mittel zur Umwandlung eines Rotationsmoments in die axial gerichtete Kraft aufweist, wobei das Mittel zur Umwandlung so ausgestaltet ist, dass es eine Variation eines auf die Nadel axial wirkenden Hubs bewirkt.

Das Dokument EP 1 495 782 A1 offenbart eine Vorrichtung zum Aufbringen einer Tätowierung oder von permanentem Make-up, mit einem Einwegmodul, welches lösbar an einem Handstück angeordnet ist, einer Nadel, die mit einem Ende in einem Nadelschaft gehalten wird, die bewegbar in einer an dem Einwegmodul gebildeten Nadelführung gelagert ist und die mit einem anderen Ende durch eine Nadeldüsenöffnung zum Ausbringen eines Farbstoffs verläuft, und einem an den Nadelschaft zum Vor- und Zurückbewegen der Nadel gekoppelten und von mehreren Bauteilen gebildeten Antriebsmechanismus, wobei die mehreren Bauteile des Antriebsmechanismus einen Antrieb und Kopplungsmittel zum Koppeln des Antriebs an den Nadelschaft umfassen. Zumindest ein Teil der mehreren Bauteile des Antriebsmechanismus ist gemäß dem Dokument EP 1 495 782 A1 in dem Einwegmodul angeordnet und zusammen mit dem Einwegmodul von dem Handstück abnehmbar.

In dem Dokument US 2018 / 369553 A1 ist eine Mikronadelmaschine zum Tätowieren offenbart, die eine im Wesentlichen radialsymmetrische äußere Form aufweist.

Das Dokument EP 1 958 659 A1 betrifft ein Antriebsmodul für eine Vorrichtung zum lokalen Aufstechen einer menschlichen oder einer tierischen Haut mit einer Antriebseinrichtung, die konfiguriert ist, eine Antriebsdrehbewegung zu erzeugen, und einem an die Antriebseinrichtung gekoppelten Wandlungsmechanismus, der konfiguriert ist, die Antriebsbewegung in eine auf eine die Haut lokal aufstechende Nadeleinrichtung einkoppelbare Vorwärts- / Rückwärtsbewegung zu wandeln, und der ein freilaufendes Funktionsbauteil umfasst, wobei das Funktionsbauteil an eine eine Taumel- oder Kippbewegung des Funktionsbauteils zulassende, magnetische Verdrehsicherung gekoppelt ist, mit der eine einem Verdrehen des Funktionsbauteils entgegenwirkende, magnetische Rückhaltekraft zur Verfügung gestellt ist.

### Zusammenfassung

Aufgabe der Erfindung ist es, ein Antriebssystem sowie ein Antriebsmodul für ein Handgerät zum lokalen Aufstechen einer menschlichen oder einer tierischen Haut sowie ein Handgerät anzugeben, die flexibel an unterschiedliche Anwendungsbedingungen anpassbar sind.

Zur Lösung sind ein Antriebssystem sowie ein Antriebsmodul für ein Handgerät zum lokalen Aufstechen einer menschlichen oder einer tierischen Haut nach den Ansprüchen 1 und 12 geschaffen. Weiterhin ist ein Handgerät zum lokalen Aufstechen einer menschlichen oder einer tierischen Haut nach Anspruch 13 geschaffen. Ausgestaltungen sind Gegenstand von abhängigen Unteransprüchen.

Nach einem Aspekt ist ein Antriebssystem für ein Handgerät zum lokalen Aufstechen einer menschlichen oder einer tierischen Haut geschaffen, welches eine Antriebseinrichtung, die eingerichtet ist, eine drehende Antriebsbewegung bereitzustellen, sowie einen Wandlungsmechanismus aufweist, der zum Abgreifen der drehenden Antriebsbewegung an die Antriebseinrichtung koppelt und eingerichtet ist, ausgehend von der drehenden Antriebsbewegung eine lineare Vorwärts- und Rückwärtsbewegung bereitzustellen. Der Wandlungsmechanismus weist Folgendes auf: eine Taumelscheibenanordnung, die an einer Lagereinrichtung angeordnet ist und über ein Drehgelenk an ein Gelenkbauteil koppelt, welches von der Lagereinrichtung aufgenommen ist; eine an der Taumelscheibenanordnung gebildete Taumelscheibe, welche im Betrieb aufgrund der drehenden Antriebsbewegung eine Taumelbewegung ausführt; einen Abgriff, welcher an die Taumelscheibe koppelt und eingerichtet ist, mittels Zusammenwirken mit der Taumelscheibe die lineare Vorwärts- und Rückwärtsbewegung zum Abgreifen bereitzustellen; und ein Einstellbauteil, welches an die Taumelscheibenanordnung koppelt und verlagerbar angeordnet ist, derart, dass zum Einstellen eines Hubs der an dem Abgriff bereitgestellten linearen Vorwärts- und Rückwärtsbewegung eine Neigung der Taumelscheibe zur Längsrichtung mittels Verlagern des Einstellbauteils veränderbar ist.

Nach einem weiteren Aspekt ist ein Antriebsmodel für ein Handgerät zum lokalen Aufstechen einer menschlichen oder einer tierischen Haut mit dem Antriebssystem geschaffen, welches an ein Nadelmodul mit einer Stecheinrichtung zum lokalen Hautaufstechen koppelbar ist. Weiterhin ist ein Handgerät zum lokalen Aufstechen einer menschlichen oder einer tierischen Haut mit dem Antriebsmodul und einem hieran gekoppelten Nadelmodul geschaffen.

Mit Hilfe des vorgeschlagenen Antriebssystems kann für verschiedene Anwendungen beim lokalen Aufstechen der Haut der Hub der linearen Vorwärts- und Rückwärtsbewegung flexibel bereitgestellt werden. Bei dem Handgerät zum lokalen Aufstechen der Haut, welches auch als Hautstechgerät bezeichnet werden kann, wird die lineare Vorwärts- und Rückwärtsbewegung, welche mit dem Antriebssystem bereitgestellt ist, auf die Stecheinrichtung eingekoppelt, welche hierauf vor- und zurückbewegt wird. Mit Hilfe des vorgeschlagenen Antriebssystems kann ein flexibel einstellbarer Hub für die lineare Vorwärts- und Rückwärtsbewegung bereitgestellt werden. Hierzu ist das Einstellbauteil, welches an die Taumelscheibenanordnung koppelt, verlagerbar, beispielsweise schwenkbar, um die Neigung oder Schrägstellung der Taumelscheibe zur Längsrichtung einzustellen, also einen Anstellwinkel. Dieses bewirkt eine Änderung des Hubs der mittels des Wandlungsmechanismus bereitgestellten linearen Vorwärts- und Rückwärtsbewegung, welche bei dem Handgerät zum lokalen Aufstechen der Haut zum Einkoppeln auf die Stecheinrichtung abgegriffen werden kann.

Zum Ändern der Neigung der Taumelscheibe zur Längsrichtung mittels Verlagern des Einstellbauteils kann eine Lagerung des Einstellbauteils in der Längsrichtung verlagerbar sein.

Die Antriebseinrichtung kann mittels eines elektrischen Motors gebildet sein. Die Wiederholfrequenz, mit der die lineare Vorwärts- und Rückwärtsbewegung bereitgestellt ist, kann zwischen etwa 0,5 und etwa 200 Hz, alternativ zwischen etwa 10 und etwa 200 Hz oder zwischen etwa 40 und etwa 200 Hz betragen.

Bei dem Handgerät kann das Nadelmodul mit zugeordneter Stecheinrichtung, welches als solches in verschiedenen Ausführungsformen bekannt ist, als Einwegmodul ausgeführt sein. Bei dem Nadelmodul können ein oder mehrere Bauteile aus einem Kunststoffmaterial hergestellt sein, beispielsweise mittels Spritzgießen. Insbesondere bei der Ausführung als Einwegmodul ist das Nadelmodul lösbar an dem Antriebsmodul angeordnet. Mit Hilfe des Antriebsmoduls wird die lineare Vorwärts- und Rückwärtsbewegung bereitgestellt, welche als Antriebsbewegung auf die Stecheinrichtung des Nadelmoduls abgegriffen wird, um ein wiederholtes lokales Aufstechen der Haut ausführen zu können.

Das Drehgelenk kann zum Beispiel als Kugelgelenk ausgeführt sein.

Das Einstellbauteil kann an der Taumelscheibenanordnung schwenkbar gelagert sein. Beispielsweise kann die Lagerung des Einstellbauteils an der Taumelscheibenanordnung mit Hilfe eines Lagerzapfens und einer zugeordneten Lageröffnung gebildet sein, in welcher der Lagerzapfen angeordnet ist. Das Einstellbauteil kann dann in gegenüberliegenden Endbereichen des Einstellbauteils jeweils schwenkbar gelagert sein.

Das Einstellbauteil kann mit einem Flachbauteil gebildet sein, dessen Flachseiten sich in der Längsrichtung erstrecken. Das Flachbauteil kann mit einem Scheibenbauteil gebildet sein. In einer Ausführungsform ist das Flachbauteil an der Bauteillagerung sowie an der Taumelscheibenanordnung schwenkbar oder drehbar gelagert.

Das Einstellbauteil kann an einer Bauteillagerung verlagerbar angeordnet ist, zum Beispiel schwenkbar, derart, dass zum Einstellen des Hubs der an dem Abgriff bereitgestellten linearen Vorwärts- und Rückwärtsbewegung die Neigung der Taumelscheibe zur Längsrichtung mittels Verlagern des Einstellbauteils veränderbar ist, wobei hierzu die Bauteillagerung in der Längsrichtung verlagerbar ist.

Das Einstellbauteil kann an der Bauteillagerung schwenkbar angeordnet sein, derart, dass zum Einstellen des Hubs der an dem Abgriff bereitgestellten linearen Vorwärts- und Rückwärtsbewegung die Neigung der Taumelscheibe zur Längsrichtung mittels Schwenken des Einstellbauteils veränderbar ist.

Das Einstellbauteil kann mittels eines Festkörpergelenks verlagerbar angeordnet sein. Zum Einstellen des Hubs der an dem Abgriff bereitgestellten linearen Vorwärts- und Rückwärtsbewegung ist die Neigung der Taumelscheibe zur Längsrichtung mittels Verlagern des Einstellbauteils veränderbar, wobei hierzu eine Lagerung des Festkörpergelenks in der Längsrichtung verlagerbar ist.

Die Bauteillagerung kann einen Abstand zwischen einer Drehgelenksmitte des Drehgelenks und der Bauteillagerung verändernd verlagerbar sein. Ist das Drehgelenk als Kugelgelenk ausgeführt, kann der Kugelkopf einstückig an dem in der Lagereinrichtung aufgenommenen Lagerbauteil gebildet sein, zum Beispiel in einem distalen Lagerbauteil. Die Taumelscheibenanordnung ist bei diesem Beispiel an dem Kugelkopf den Freiheitsgraden eines Kugelgelenks entsprechend beweglich aufgenommen. Bei dieser oder anderen Ausführungsformen kann die Taumelscheibenanordnung ein inneres Bauteil aufweisen, welches auf dem Kugelkopf sitzt. Ein den Kugelkopf aufnehmender Abschnitt des inneren Bauteils kann über eine Drehentkopplung, die mit einem Kugellager gebildet sein kann, an die Taumelscheibe koppeln.

Beim Einstellen der Neigung der Taumelscheibe zur Längsrichtung, welche ihrerseits mit einer Axialrichtung einer Antriebswelle der Antriebseinrichtung zusammenfallen kann, wird eine Ebene quer zur Längsrichtung mehr oder weniger geneigt, welche sich durch die Drehgelenksmitte erstreckt, insbesondere die Kugelkopfmitte des Kugelgelenks.

Die Bauteillagerung kann einen Lagerzapfen aufweisen, welcher drehbar in einer zugeordneten Lagerbuchse angeordnet ist. Die Lagerbuchse kann an dem Einstellbauteil vorgesehen sein.

Die Bauteillagerung kann einer Verlagerung der Antriebseinrichtung in Längsrichtung folgend verlagerbar sein. Eine Verlagerung der Antriebseinrichtung, zum Beispiel eines hiervon umfassten elektrischen Motors mit Antriebswelle, in Längsrichtung bewirkt oder erzwingt eine Verlagerung, zum Beispiel (Verschieben) der Bauteillagerung, so dass sich die Neigung der Taumelscheibe verstellt. Hierbei können die Bauteillagerung und die Antriebseinrichtung einem fixen Abstand in Längsrichtung entsprechend miteinander verbunden sein, beispielsweise derart, dass ein auf der Antriebswelle der Antriebseinrichtung sitzendes Bauteil die Bauteillagerung bereitstellt oder an diese fest koppelt, an welche das Einstellbauteil schwenkbar koppelt.

Die Bauteillagerung kann an einem in Längsrichtung relativ zur Antriebseinrichtung verlagerbaren Bauteil angeordnet sein, zum Beispiel in einem die Antriebseinrichtung aufnehmenden Gehäuse. Bei dieser Ausführungsform kann die Antriebseinrichtung selbst ortsfest angeordnet sein. Relativ zur Antriebseinrichtung wird das verlagerbare Bauteil verlagert, um die Bauteillagerung in Längsrichtung zu verlagern, insbesondere zu verschieben, um so die Neigung der Taumelscheibe einzustellen. Das verlagerbare Bauteil, an dem die Bauteillagerung gebildet ist, kann hierbei eine Relativverlagerung in Bezug auf die Lagereinrichtung ausführen.

Die Antriebseinrichtung und der Wandlungsmechanismus können in einem Gehäuse angeordnet sein, wobei die Lagereinrichtung in dem Gehäuse gehäusefest angeordnet ist.

Die Lagereinrichtung kann eine in Bezug auf die Antriebseinrichtung proximale Lagereinrichtung und eine in Bezug auf die Antriebseinrichtung distale Lagereinrichtung aufweisen, die in einer Längsrichtung von der proximalen Lagereinrichtung beabstandet ist. Sind die Lagereinrichtungen ortsfest im Gehäuse vorgesehen, ist der Abstand in Längsrichtung zwischen der proximalen und der distalen Lagereinrichtung fest. Eine solche Ausführungsform kann auch bei anderen Ausgestaltungen vorgesehen sein.

Die Taumelscheibenanordnung kann zwischen der proximalen und der distalen Lagereinrichtung angeordnet sein.

Die Längsrichtung kann mit der Axialrichtung der Antriebswelle zusammenfallen.

Die vorangehend beschriebenen Ausgestaltungen können bei dem Antriebsmodul sowie bei dem Handgerät entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgeräts zum lokalen Aufstechen einer menschlichen oder einer tierischen Haut mit einem Antriebsmodul und einem hieran gekoppelten Nadelmodul;
- Fig. 2: eine perspektivische Darstellung eines Antriebssystems für das Handgerät.
- Fig. 3: eine perspektivische Darstellung des Antriebssystems aus Fig. 2 im Schnitt;
- Fig. 4: eine perspektivische Darstellung des Antriebssystems aus Fig. 3 mit einer anderen Betriebsstellung im Schnitt;
- Fig. 5: eine perspektivische Darstellung des Antriebssystems aus Fig. 3 mit einer weiteren Betriebsstellung im Schnitt;
- Fig. 6: eine perspektivische Darstellung des Antriebssystems aus Fig. 3 mit noch einer geänderten Betriebsstellung im Schnitt;
- Fig. 7: eine schematische funktionelle Darstellung für Elemente des Antriebssystems aus Fig. 3 und Fig. 4 mit einer Hubeinstellung A;
- Fig. 8: eine schematische funktionelle Darstellung für Elemente des Antriebssystems aus Fig. 5 und Fig. 6 mit einer Hubeinstellung B;
- Fig. 9: eine schematische funktionelle Darstellung für Elemente einer anderen Ausführung eines Antriebssystems mit einer Hubeinstellung A und
- Fig. 10: eine schematische funktionelle Darstellung für Elemente der anderen Ausführung nach Fig. 9 mit einer Hubeinstellungen B, die von der Hubeinstellung A verschieden ist.

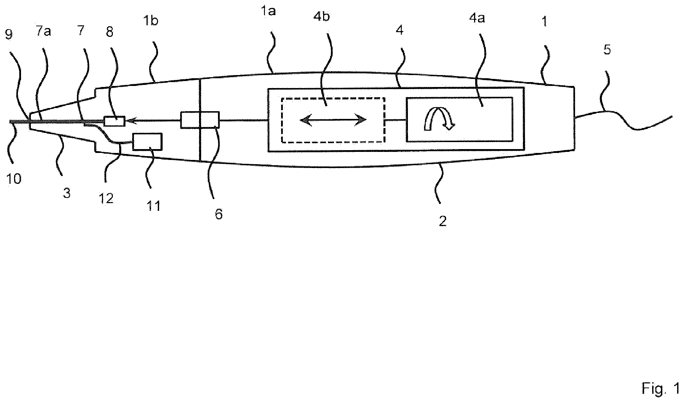

Fig. 1 zeigt eine schematische Darstellung eines Handgeräts zum wiederholten lokalen Aufstechen einer menschlichen oder einer tierischen Haut (Hautstechgerät) mit einem Gehäuse 1. Das Gehäuse 1 ist im gezeigten Beispiel modulartig aufgebaut mit einem Antriebsmodul-Gehäuse 1a eines Antriebsmoduls 2 und einem Nadelmodul-Gehäuse 1b eines Nadelmoduls 3, welches lösbar an das Antriebsmodul 2 koppelt.

In dem Antriebsmodul-Gehäuse 1a ist ein Antriebssystem 4 vorgesehen, welches in der dargestellten Ausführungsform eine Antriebseinrichtung 4a mit einem Motor sowie einen Wandlungsmechanismus 4b aufweist. Mittels des Motors wird bei dem Beispiel eine drehende Antriebsbewegung (Antriebskraft) bereitgestellt, die mit Hilfe des Wandlungsmechanismus 4b in eine in linearer Richtung wirkende Antriebskraft in Längsrichtung (Vor- und Zurückbewegung) des Gehäuses 1 gewandelt wird, sei es zum Ausführen eines Einzelhubs oder für eine repetitive Bewegung.

Zur Energieversorgung kann das Handgerät über eine Zuleitung 5 an eine Versorgungsspannung angeschlossen werden. Die Zuleitung 5 kann ergänzend Kabel umfassen, über die Steuersignale zwischen einem Steuergerät (nicht dargestellt) und dem Handgerät übertragen werden.

Die vom Antriebssystem 4 maschinell erzeugte Antriebskraft wird über eine Kopplungseinrichtung 6 auf eine Stecheinrichtung 7 mit einem Stechwerkzeug 7a gegeben, welches in einer Werkzeugaufnahme 8 angeordnet ist. Im Fall der Verwendung einer oder mehrerer Nadeln als Stechwerkzeug 7a kann es sich bei der Werkzeugaufnahme 8 um einen Nadelschaft handeln, der in dem Nadelmodul-Gehäuse 1b in einer zugeordneten Führung angeordnet ist, so dass die repetitive oder zyklische Antriebskraft in linearer Richtung für eine Vor- und Rückwärtsbewegung von Werkzeugaufnahme 8 mit Stechwerkzeug 7a umgesetzt werden kann. Das Stechwerkzeug 7a ist durch eine Gehäuseöffnung 9 frontseitig an dem Nadelmodul-Gehäuse 1b geführt, so dass zumindest in einer ausgefahrenen Stellung eine Stechwerkzeugspitze 10 außerhalb des Nadelmodul-Gehäuses 1b angeordnet ist, um die zu bearbeitende Haut aufzustechen. Die Gehäuseöffnung 9 ist bei der gezeigten Ausführungsform am vorderen Ende des Nadelmodul-Gehäuses 1b an dem Nadelmodul 3 gebildet, welches lösbar an dem Antriebsmodul-Gehäuse 1a angeordnet ist.

Bei der gezeigten Ausführungsform des Handgeräts ist in dem Gehäuse 1 weiterhin ein Reservoir 11 vorgesehen, welches zur Aufnahme einer in die Haut einzubringenden Substanz dient, zum Beispiel einer Farbe im Fall des Herstellens eines Tattoos oder von permanentem Makeup. Über einen Ausfluss 12 wird die Substanz an das Stechwerkzeug 7a herangeführt, um so an der Stechwerkzeugspitze 10 die Substanz zum Einbringen in die Haut bereitzustellen. Alternativ kann das Reservoir 11 auch im Antriebsmodul-Gehäuse 1a oder außerhalb des Gehäuses 1 angeordnet sein. Verschiedene Ausgestaltungen für ein solches Reservoir und das Bereitstellen der einzubringenden Substanz im Bereich des Stechwerkzeugs 7 sind als solche bekannt, beispielsweise auch unter Verwendung einer Pumpeinrichtung an dem Reservoir 11.

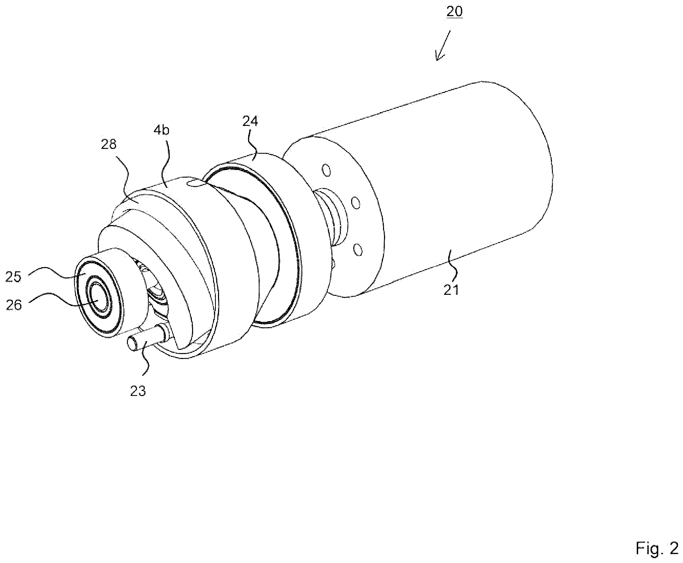

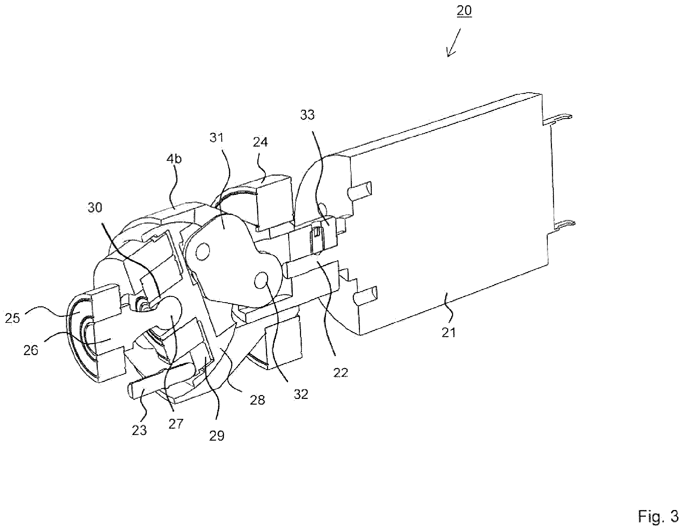

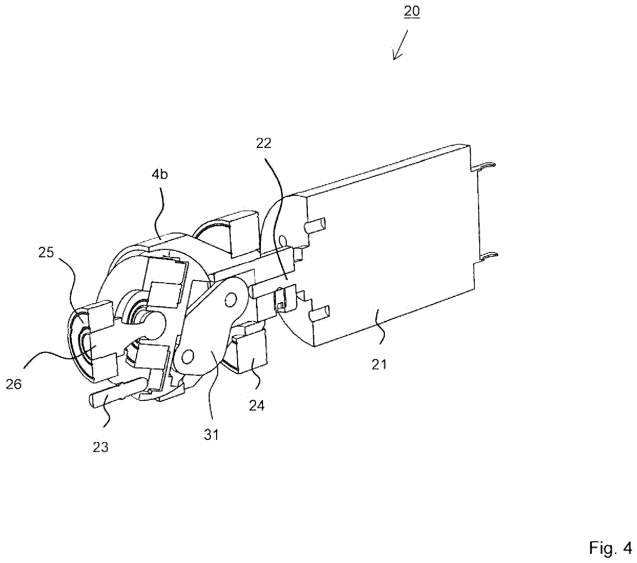

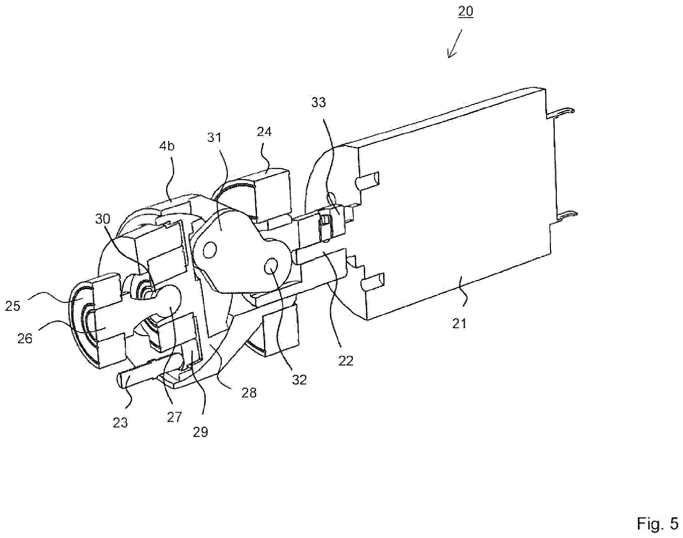

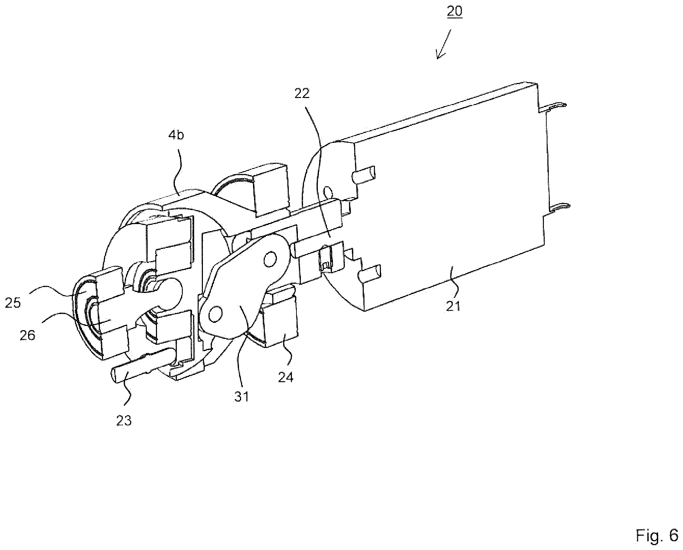

Fig. 2 zeigt eine schematische perspektivische Darstellung eines Antriebssystems 20, welches beispielsweise für das Antriebsmodul 2 nutzbar ist. Die Fig. 3 bis 6 zeigen jeweilige Schnittdarstellungen des Antriebssystems 20 mit unterschiedlichen Betriebsstellungen.

Mit Hilfe eines elektrischen Motors 21 wird über eine Antriebswelle 22 eine drehende Antriebsbewegung bereitgestellt, die von dem Wandlungsmechanismus 4b abgegriffen und in eine lineare Vor- und Rückwärtsbewegung gewandelt wird, welche über einen Abgriff 23 abgegriffen werden kann, um diese lineare Vorwärts- und Rückwärtsbewegung als Antriebsbewegung auf eine Stecheinrichtung des Nadelmoduls 3 einzukoppeln. Der Abgriff 23 kann Teil der Kopplungseinrichtung 6 sein.

Bei dem Wandlungsmechanismus 4b sind in Bezug auf den elektrischen Motor 21 eine proximale und eine distale Lagereinrichtung 24, 25 vorgesehen, die in einer Ausführungsform in einem Gehäuse des Antriebsmoduls 2 ortsfest oder gehäusefest gelagert sein können. An der distalen Lagereinrichtung 25 ist ein Lagerbauteil 26 mit einem Kugelkopf 27 gelagert, der bei dem gezeigten Beispiel einstückig angeformt ist. Auf dem Kugelkopf 27 sitzt eine Taumelscheibenanordnung 28 mit einer Taumelscheibe 29. Der Kugelkopf 27 bildet zusammen mit einem inneren Bauteil 30 der Taumelscheibenanordnung 28 ein Kugelgelenk.

An die Taumelscheibenanordnung 28 koppelt ein Einstellbauteil 31, welches sowohl an der Taumelscheibenanordnung 28 wie auch an einer Bauteillagerung 32 schwenkbar gelagert ist. Bei der dargestellten Ausführungsform ist das Einstellbauteil 31 mit einem Scheiben- oder Flachbauteil gebildet. Über ein Kopplungsbauteil 33 ist die Bauteillagerung 32 mit der Antriebswelle 22 fest verbunden. Aufgrund der drehenden Antriebsbewegung, welche über die Antriebswelle 22 bereitgestellt ist, drehen sich das Kopplungsbauteil 33 sowie das hieran koppelnde Einstellbauteil 31 wie auch Teile der Taumelscheibenanordnung 28 relativ zu der proximalen und der distalen Lagereinrichtung 24, 25, wodurch die Taumelscheibe 29 eine taumelnde Bewegung ausführt, die das Abgreifen der linearen Vor- und Rückwärtsbewegung an dem Abgriff 23 ermöglicht.

Mittels Verlagern der Bauteillagerung 32 in Längsrichtung kann der Abstand der Bauteillagerung 32 zur Mitte des Kugelkopfes 27 verändert werden, was ein Verändern der Neigung oder Schrägstellung der Taumelscheibe 29 zur Längsrichtung bewirkt, so dass hierdurch ein Hub der linearen Vorwärts- und Rückwärtsbewegung einstellbar ist.

Zum Verlagern der Bauteillagerung 32 in Längsrichtung können unterschiedliche Ausgestaltungen vorgesehen sein. In einem Beispiel kann der elektrische Motor 21 in Längsrichtung verlagert werden. Über die Antriebswelle 22 und das hiermit fest verbundene Kopplungsbauteil 33 wird dann die Bauteillagerung 32 ebenso in Längsrichtung verlagert. Alternativ kann der elektrische Motor 21 ortsfest im Gehäuse des Antriebsmoduls 2 aufgenommen sein. Das mit der Antriebswelle 22 drehfest verbundene Kopplungsbauteil 33 kann dann in Längsrichtung verlagert werden, um die Bauteillagerung 32 zu verschieben. Hierfür kann ein Antrieb (nicht dargestellt) vorgesehen sein, um das Kopplungsbauteil 33 zu verlagern.

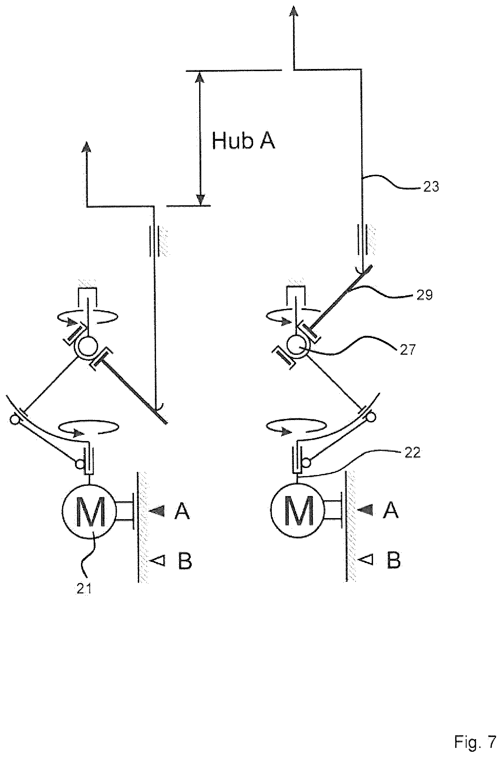

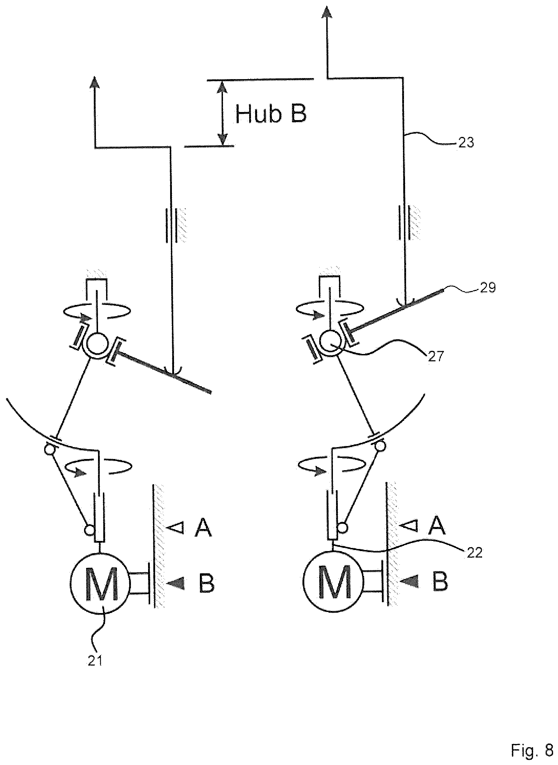

Fig. 7 und 8 zeigen schematische funktionelle Darstellungen für Elemente des Antriebssystems aus Fig. 2 bis 6 mit unterschiedlichen Hubeinstellungen A, B. Für gleiche Merkmale werden dieselben Bezugszeichen wie in den Fig. 2 bis 6 verwendet. In den Fig. 7 und 8 sind jeweils zwei unterschiedliche Drehstellungen für die Taumelscheibe 29 gezeigt, deren taumelnde Bewegung zu dem Hub A (Fig. 7) oder dem Hub B (Fig. 8) für die lineare Vorwärts- und Rückwärtsbewegung führt. Bewirkt werden die unterschiedlichen Hübe durch die verschiedenen Schrägstellungen der Taumelscheibe 29. Bei dem gezeigten Beispiel wird der elektrische Motor 21 verlagert (Motorverschiebung).

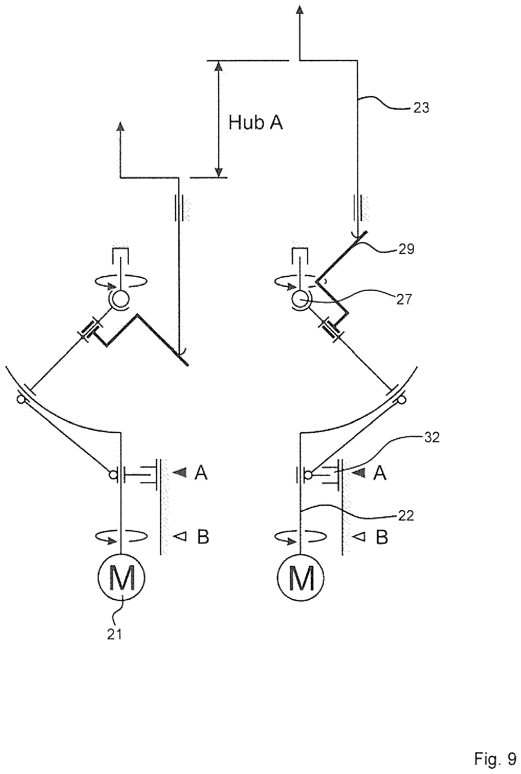

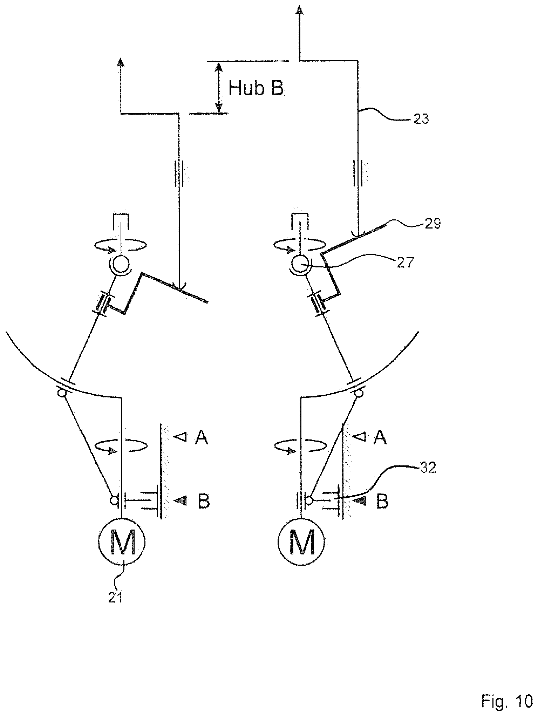

Fig. 9 und 10 zeigen schematische funktionelle Darstellungen für Elemente einer anderen Ausführung mit unterschiedlichen Hubeinstellungen A, B. Für gleiche Merkmale werden dieselben Bezugszeichen wie in den Fig. 2 bis 6 verwendet. In den Fig. 9 und 10 sind jeweils zwei unterschiedliche Drehstellungen für die Taumelscheibe 29 gezeigt, deren taumelnde Bewegung zu dem Hub A (Fig. 9) oder dem Hub B (Fig. 10) für die lineare Vorwärts- und Rückwärtsbewegung führt. Bewirkt werden die unterschiedlichen Hübe durch die verschiedenen Schrägstellungen der Taumelscheibe 29. Bei dem gezeigten alternativen Beispiel verbleibt der elektrische Motor 21 ortsfest, wenn zur Hubeinstellung die Bauteillagerung 32 (relativ zum Kugelkopf 27) verlagert wird. Zum Implementieren kann zum Beispiel eine in Axialrichtung verschiebliche Gehäuse- / Einstellhülse mit umlaufender Nut auf der Innenseite vorgesehen sein, in der eine Verschiebenocke auf einer rotierenden Baugruppe in der Nut umläuft und mittels Hülsenverschiebung bezüglich der rotierenden Baugruppe verlagert wird.

## Patentansprüche

1. Antriebssystem für ein Handgerät zum lokalen Aufstechen einer menschlichen oder einer tierischen Haut, mit:
- einer Antriebseinrichtung (21), die eingerichtet ist, eine drehende Antriebsbewegung bereitzustellen; und
- einem Wandlungsmechanismus (4b), welcher zum Abgreifen der drehenden Antriebsbewegung an die Antriebseinrichtung (21) koppelt und eingerichtet ist, ausgehend von der drehenden Antriebsbewegung eine lineare Vorwärts- und Rückwärtsbewegung bereitzustellen, der Wandlungsmechanismus (4b) aufweisend:
- eine Taumelscheibenanordnung (28), die an einer Lagereinrichtung (24, 25) angeordnet ist;
- eine an der Taumelscheibenanordnung (28) gebildete Taumelscheibe (29), welche im Betrieb aufgrund der drehenden Antriebsbewegung eine Taumelbewegung ausführt;
- einen Abgriff (23), welcher an die Taumelscheibe (29) koppelt und eingerichtet ist, mittels Zusammenwirken mit der Taumelscheibe (29) die lineare Vorwärts- und Rückwärtsbewegung zum Abgreifen bereitzustellen; und
- ein Einstellbauteil (31), welches an die Taumelscheibenanordnung (28) koppelt und verlagerbar angeordnet ist, derart, dass zum Einstellen eines Hubs der an dem Abgriff (23) bereitgestellten linearen Vorwärts- und Rückwärtsbewegung eine Neigung der Taumelscheibe (29) zur Längsrichtung mittels Verlagern des Einstellbauteils (31) veränderbar ist;
**gekennzeichnet durch** ein distales Gelenkbauteil (26), welches von der Lagereinrichtung (25) aufgenommen ist und an das die Taumelscheibenanordnung (28) über ein Drehgelenk koppelt.

2. Antriebssystem nach Anspruch 1, **dadurch gekennzeichnet, dass** das Einstellbauteil (31) an der Taumelscheibenanordnung (28) schwenkbar gelagert ist.

3. Antriebssystem nach Anspruch 1 oder 2, **dadurch gekennzeichnet, dass** das Einstellbauteil (31) mit einem Flachbauteil gebildet ist, dessen Flachseiten sich in der Längsrichtung erstrecken.

4. Antriebssystem nach mindestens einem der vorangehenden Ansprüche, **dadurch gekennzeichnet, dass** das Einstellbauteil (31) an einer Bauteillagerung (32) verlagerbar angeordnet ist, derart, dass zum Einstellen des Hubs der an dem Abgriff (23) bereitgestellten linearen Vorwärts- und Rückwärtsbewegung die Neigung der Taumelscheibe (29) zur Längsrichtung mittels Verlagern des Einstellbauteils (31) veränderbar ist, wobei hierzu die Bauteillagerung (32) in der Längsrichtung verlagerbar ist.

5. Antriebssystem nach mindestens einem der vorangehenden Ansprüche, soweit auf Anspruch 4 rückbezogen, **dadurch gekennzeichnet, dass** die Bauteillagerung (32) einen Abstand zwischen einer Drehgelenksmitte des Drehgelenks und der Bauteillagerung (32) verändernd verlagerbar ist.

6. Antriebssystem nach mindestens einem der vorangehenden Ansprüche, soweit auf Anspruch 4 rückbezogen, **dadurch gekennzeichnet, dass** die Bauteillagerung (32) einen Lagerzapfen aufweist, welcher drehbar in einer zugeordneten Lagerbuchse angeordnet ist.

7. Antriebssystem nach mindestens einem der vorangehenden Ansprüche, soweit auf Anspruch 4 rückbezogen, **dadurch gekennzeichnet, dass** die Bauteillagerung (32) einer Verlagerung der Antriebseinrichtung (21) in Längsrichtung folgend verlagerbar ist.

8. Antriebssystem nach mindestens einem der vorangehenden Ansprüche, soweit auf Anspruch 4 rückbezogen, **dadurch gekennzeichnet, dass** die Bauteillagerung (32) an einem in Längsrichtung relativ zur Antriebseinrichtung (21) verlagerbaren Bauteil angeordnet ist.

9. Antriebssystem nach mindestens einem der vorangehenden Ansprüche, **dadurch gekennzeichnet, dass** die Antriebseinrichtung (21) und der Wandlungsmechanismus (4b) in einem Gehäuse angeordnet sind, wobei die Lagereinrichtung (24, 25) in dem Gehäuse gehäusefest angeordnet ist.

10. Antriebssystem nach mindestens einem der vorangehenden Ansprüche, **dadurch gekennzeichnet, dass** die Lagereinrichtung eine in Bezug auf die Antriebseinrichtung (21) proximale Lagereinrichtung (24) und eine in Bezug auf die Antriebseinrichtung (21) distale Lagereinrichtung (25) aufweist, die in einer Längsrichtung von der proximalen Lagereinrichtung (24) beabstandet ist.

11. Antriebssystem nach Anspruch 10, **dadurch gekennzeichnet, dass** die Taumelscheibenanordnung (28) zwischen der proximalen und der distalen Lagereinrichtung (24, 25) angeordnet ist.

12. Antriebsmodul für ein Handgerät zum lokalen Aufstechen einer menschlichen oder einer tierischen Haut, welches ein Antriebssystem nach mindestens einem der vorangehenden Ansprüche aufweist und an ein Nadelmodul (3) mit einer Stecheinrichtung (7) zum lokalen Hautaufstechen koppelbar ist.

13. Handgerät zum lokalen Aufstechen einer menschlichen oder einer tierischen Haut, aufweisend ein Antriebsmodul nach Anspruch 12 und ein hieran gekoppeltes Nadelmodul (3) mit einer Stecheinrichtung (7) zum lokalen Hautaufstechen.

## Claims

1. A drive system for a hand-held device for locally piercing human or animal skin, with:
- a drive means (21) which is configured to provide a rotary drive movement; and
- a conversion mechanism (4b) which is coupled to the drive means (21) for tapping the rotary drive movement and is configured to provide a linear forwards and backwards movement emanating from the rotary drive movement, the conversion mechanism (4b) having:
- a wobble plate assembly (28) which is disposed on a bearing means (24, 25);
- a wobble plate (29) formed on the wobble plate assembly (28) which, during operation, executes a wobbling motion due to the rotary drive movement;
- a tap (23) which is coupled to the wobble plate (29) and is configured to provide the linear forwards and backwards movement for tapping by means of cooperation with the wobble plate (29); and
- an adjustment component (31) which is coupled to the wobble plate assembly (28) and is displaceably disposed in a manner such that in order to adjust a stroke of the linear forwards and backwards movement provided at the tap (23), an inclination of the wobble plate (29) with respect to the longitudinal direction is variable by displacing the adjustment component (31);
**characterized by** a distal joint component (26) which is received by the bearing means (25) and is coupled to the wobble plate assembly (28) via a rotary joint.

2. The drive system as claimed in claim 1, **characterized in that** the adjustment component (31) is pivotably mounted on the wobble plate assembly (28).

3. The drive system as claimed in claim 1 or claim 2, **characterized in that** the adjustment component (31) is formed with a flat component the flat sides of which extend in the longitudinal direction.

4. The drive system as claimed in at least one of the preceding claims, **characterized in that** the adjustment component (31) is displaceably disposed on a component bearing (32) in a manner such that, in order to adjust the stroke of the linear forwards and backwards movement provided at the tap (23), the inclination of the wobble plate (29) with respect to the longitudinal direction is variable by displacing the adjustment component (31), wherein for this purpose, the component bearing (32) is displaceable in the longitudinal direction.

5. The drive system as claimed in at least one of the preceding claims insofar as it relates to claim 4, **characterized in that** the component bearing (32) is displaceable so as to vary a distance between a rotary joint centre of the rotary joint and the component bearing (32).

6. The drive system as claimed in at least one of the preceding claims insofar as it relates to claim 4, **characterized in that** the component bearing (32) has a bearing journal which is rotatably disposed in an associated bearing bush.

7. The drive system as claimed in at least one of the preceding claims insofar as it relates to claim 4, **characterized in that** the component bearing (32) is displaceable following a displacement of the drive means (21) in the longitudinal direction.

8. The drive system as claimed in at least one of the preceding claims insofar as it relates to claim 4, **characterized in that** the component bearing (32) is disposed on a component which is displaceable in the longitudinal direction relative to the drive means (21).

9. The drive system as claimed in at least one of the preceding claims, **characterized in that** the drive means (21) and the conversion mechanism (4b) are disposed in a housing, wherein the bearing means (24, 25) is arranged in the housing fixed relative to the housing.

10. The drive system as claimed in at least one of the preceding claims, **characterized in that** the bearing means has a bearing means (24) which is proximal with respect to the drive means (21) and a bearing means (25) which is distal with respect to the drive means (21) and is at a distance from the proximal bearing means (24) in a longitudinal direction.

11. The drive system as claimed in claim 10, **characterized in that** the wobble plate assembly (28) is disposed between the proximal and the distal bearing means (24, 25).

12. A drive module for a hand-held device for locally piercing human or animal skin, which has a drive system as claimed in at least one of the preceding claims and which can be coupled to a needle module (3) with a piercing means (7) for local skin piercing.

13. A hand-held device for locally piercing human or animal skin, having a drive module as claimed in claim 12 and a needle module (3) with a piercing means (7) coupled thereto for locally piercing the skin.

## Revendications

1. Système d'entraînement pour un dispositif portatif pour piquer localement la peau humaine ou animale, comprenant :
- un dispositif d'entraînement (21), qui est conçu pour fournir un mouvement d'entraînement rotatif ; et
- un mécanisme de conversion (4b), qui est couplé au dispositif d'entraînement (21) afin de capter le mouvement d'entraînement rotatif et est configuré pour fournir un mouvement linéaire d'avant en arrière, à partir du mouvement d'entraînement rotatif, le mécanisme de conversion (4b) présentant :
- un ensemble de plateau oscillant (28), qui est disposé sur un ensemble de palier (24, 25);
- un plateau oscillant (29) formé sur l'ensemble de plateau oscillant (28) qui, en utilisation, provoque un mouvement oscillant dû à l'exécution du mouvement d'entraînement rotatif;
- un capteur (23), qui est couplé au plateau oscillant (29) et agencé pour, par coopération avec le plateau oscillant (29), fournir un mouvement linéaire vers l'avant et fournir un mouvement inverse pour le taraudage ; et
- un composant de réglage (31), qui est couplé à l'ensemble de plateau oscillant (28) et est agencé de manière à pouvoir coulisser de telle sorte qu'une inclinaison du plateau oscillant (29) par rapport à la direction longitudinale puisse être modifiée en déplaçant le composant de réglage (31) afin de régler une course du mouvement linéaire d'avant en arrière prévu au niveau du capteur (23);
**caractérisé par** un composant d'articulation distal (26) qui est reçu par le dispositif de palier (25) et auquel l'agencement de plateau oscillant (28) est couplé via une articulation pivotante.

2. Système d'entraînement selon la revendication 1, **caractérisé en ce que** le composant de réglage (31) est monté de manière pivotante sur le plateau oscillant (28).

3. Système d'entraînement selon la revendication 1 ou 2, **caractérisé en ce que** le composant de réglage (31) est formé avec un composant plat dont les côtés plats s'étendent dans la direction longitudinale.

4. Système d'entraînement selon au moins une des revendications précédentes, **caractérisé en ce que** le composant de réglage (31) est agencé de manière déplaçable sur un palier de composant (32) de telle sorte que pour régler la course du mouvement linéaire vers l'avant et vers l'arrière prévu au niveau du capteur (23) l'inclinaison du plateau oscillant (29) dans la direction longitudinale puisse être modifiée en déplaçant l'élément de réglage (31), dans lequel le palier de composant (32) est à cet effet déplaçable dans la direction longitudinale.

5. Système d'entraînement selon au moins une des revendications précédentes, dans la mesure où il dépend de la revendication 4, **caractérisé en ce que** le palier de composant (32) peut être déplacé pour modifier une distance entre un centre d'articulation pivotante de l'articulation pivotante et le palier de composant (32).

6. Système d'entraînement selon au moins une des revendications précédentes, dans la mesure où il dépend de la revendication 4, **caractérisé en ce que** le palier de composant (32) présente un tourillon de palier qui est monté rotatif dans un coussinet de palier associé.

7. Système d'entraînement selon au moins une des revendications précédentes, dans la mesure où il dépend de la revendication 4, **caractérisé en ce que** le palier de composant (32) est déplaçable dans la direction longitudinale suite à un déplacement du dispositif d'entraînement (21).

8. Système d'entraînement selon au moins une des revendications précédentes, dans la mesure où il dépend de la revendication 4, **caractérisé en ce que** le palier de composant (32) est disposé sur un composant déplaçable dans la direction longitudinale par rapport au dispositif d'entraînement (21).

9. Système d'entraînement selon au moins une des revendications précédentes, **caractérisé en ce que** le dispositif d'entraînement (21) et le mécanisme de conversion (4b) sont agencés dans un carter, dans lequel le dispositif de palier (24, 25) est disposé de manière stationnaire dans le carter.

10. Système d'entraînement selon au moins une des revendications précédentes, **caractérisé en ce que** le dispositif de palier par rapport au dispositif d'entraînement (21) présente un dispositif de palier proximal (24) et par rapport aux moyens d'entraînement (21) un dispositif d'appui distal (25), qui est espacé dans une direction longitudinale du dispositif d'appui proximal (24).

11. Système d'entraînement selon la revendication 10, **caractérisé en ce que** le dispositif de plateau oscillant (28) est disposé entre le dispositif de palier proximal et le dispositif de palier distal (24, 25).

12. Module d'entraînement pour un appareil portatif pour le piquage local de la peau humaine ou animale, qui présente un système d'entraînement selon au moins une des revendications et peut être couplé à un module d'aiguille (3) avec un dispositif de piquage (7) pour le piquage local de la peau.

13. Appareil portatif pour le piquage local de la peau humaine ou animale, présentant un module d'entraînement selon la revendication 12 et un module d'aiguille couplé à celui-ci (3) avec un dispositif de piquage (7) pour le piquage local de la peau.
